Europäisches Patentamt

European Patent Office

Office européen des brevets

(11) Veröffentlichungsnummer: **0 223 878**
**B1**

(12) **EUROPÄISCHE PATENTSCHRIFT**

(45) Veröffentlichungstag der Patentschrift:
06.06.90

(51) Int. Cl.⁵: **A61B 10/00**, B01L 3/00,
G01N 1/02

(21) Anmeldenummer: 85115102.7

(22) Anmeldetag: 28.11.85

(54) Probenaufnahmegefäss für pastöses Probenmaterial und Verfahren zur Verarbeitung von pastösem Probenmaterial.

(43) Veröffentlichungstag der Anmeldung:
03.06.87 Patentblatt 87/23

(45) Bekanntmachung des Hinweises auf die Patenterteilung:
06.06.90 Patentblatt 90/23

(84) Benannte Vertragsstaaten:
AT BE CH DE FR GB IT LI LU NL SE

(56) Entgegenhaltungen:
CH-A- 474 060
DE-A- 3 427 114
DE-B- 2 835 358
US-A- 4 032 437
US-A- 4 081 356
US-A- 4 288 316
US-A- 4 293 405
US-A- 4 318 803

(73) Patentinhaber: Szabados, Andreas, Dr.med.,
Otto-Heilmann-Strasse 2, D-8022 Grünwald(DE)

(72) Erfinder: Szabados, Andreas, Dr.med.,
Otto-Heilmann-Strasse 2, D-8022 Grünwald(DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86(DE)

**Beschreibung**

Die Erfindung betrifft ein Probenaufnahmegefäß für in Flüssigkeit, insbesondere Suspendierflüssigkeit zu verteilendes pastöses Probenmaterial, insbesondere Stuhl, mit einem an einem Gefäßdeckel angebrachten, zum Gefäßboden hin offenen Probenaufnahmebecher im Gefäßinnenraum.

Ein Probenaufnahmegefäß dieser Art ist bekannt (z.B. Saarstedt-Katalog 77/78 W. Saarstedt, Rommelsdorf, 5523 Nümbrecht). Der Probenaufnahmebecher besteht aus einem am Gefäßdeckel angebrachten Löffel. Möchte man bei einem derartigen Probenaufnahmegefäß eine feine Verteilung des pastösen Probenmaterials in der Suspendierflüssigkeit erhalten, so bietet sich ein Umrühren des Löffels im Gefäßinnenraum an. Es läßt sich hiermit jedoch in vielen Fällen keine ausreichend feine Verteilung des Probenmaterials in der Flüssigkeit erreichen. Das Umrühren bedeutet einen zusätzlichen, im Falle von Stuhlproben, darüber hinaus unangenehmen Arbeitsgang.

Die Aufgabe der Erfindung liegt darin, ein Probenaufnahmegefäß bereitzustellen, welches bei einfachster Handhabung eine sehr gute Verteilung des Probenmaterials in der Flüssigkeit sicherstellt.

Die Aufgabe wird dadurch gelöst, daß die Becherwand wenigstens bereichsweise mit Sieböffnungen versehen ist, daß der Gefäßboden mit einer Erhebung ausgebildet ist, deren dem Gefäßinnenraum zugewandte Oberseite im wesentlichen komplementär zur Becherinnenseite geformt ist, und daß die Becherinnenseite bei geschlossenem Gefäß an der Oberseite der Erhebung im wesentlichen vollflächig anliegt. Beim Aufsetzen des Gefäßdeckels auf das Probenaufnahmegefäß wird demzufolge zwangsläufig das im Probenaufnahmebecher befindliche Probenmaterial durch die Sieböffnungen hindurch in die Suspendierflüssigkeit gepreßt; entsprechend der Anzahl und der Abmessung der Sieböffnungen ergibt sich eine feine Verteilung des Probenmaterials in der Suspendierflüssigkeit. Das Probenmaterial wird ohne merkliche Verluste in die Suspendierflüssigkeit gebracht, da die Erhebung nach Art eines Stempels den Becher vollständig ausfüllt. Es besteht folglich die Möglichkeit für quantitative Auswertungen, da der Probenaufnahmebecher zur Aufnahme eines definierten Probenvolumens geeignet ist.

In Weiterbildung der Erfindung wird vorgeschlagen, daß die erhebungsseitigen Sieböffnungsränder der Becherwand scharfkantig sind. Auf diese Weise wird der Zerkleinerungseffekt der Sieböffnungen beim Durchpressen des Probenmaterials durch die Sieböffnungen verstärkt. Für die nachfolgenden Messungen zu große Probenpartikel werden auf diese Weise zuverlässig zerkleinert, was durch das herkömmliche Rühren in vielen Fällen nicht erreicht wird.

Bei Stuhlproben haben die Sieböffnungen bevorzugt eine lichte Weite von etwa 0,5 bis 2 mm, vorzugsweise etwa 1 mm.

Um im Falle größerer, jedoch nicht zerkleinerbarer Partikel das Probenaufnahmegefäß dennoch abschließen zu können, wird vorgeschlagen, daß der Probenaufnahmebecher am Gefäßdeckel in Richtung zum Deckel nachgiebig gehalten ist. Alternativ oder zusätzlich hierzu kann man die Becherwand und/oder die Erhebung nachgiebig ausbilden. Diese Teile können plastisch oder elastisch nachgiebig ausgebildet sein, wobei jedoch die elastische Nachgiebigkeit zumindest bei Mehrfachverwendung des Gefäßes bevorzugt ist. Da das Probenaufnahmegefäß samt Gefäßdeckel aufgrund seiner einfachen Form kostengünstig herstellbar ist, wird man im allgemeinen jedoch das Probenaufnahmegefäß als Einweg-Teil einsetzen.

Die gewünschte elastische Nachgiebigkeit bei geringen Herstellungskosten erreicht man bevorzugt dadurch, daß man die Halterung für den Probenaufnahmebecher am Gefäßdeckel und/oder die Becherwand und/oder die Erhebung mit Polyäthylen bildet. Die Halterung ist hierbei bevorzugt stielförmig.

In einer ersten, besonders einfachen Ausführungsform der Erfindung ist die Becherwand im wesentlichen kalottenförmig gekrümmt.

In einer hierzu alternativen Ausführungsform ist die Becherwand von einem mit den Sieböffnungen versehenen Becherboden sowie einer vom Becherboden ausgehenden, im wesentlichen hohlzylindrischen Becherseitenwand gebildet. Diese, einer Kolben-Zylinder-Anordnung gleichende Ausführungsform stellt auch bei relativ großem Probenvolumen sicher, daß praktisch das gesamte Probenvolumen aus dem Becher in die Suspendierflüssigkeit gepreßt wird.

Um ein seitliches Entweichen von Probenmaterial zwischen Gefäßboden und Umfangsrand der Becherwand ohne gleichzeitige Zerkleinerung bzw. Feinverteilung dieses Probenmaterials in die Suspendierflüssigkeit zu vermeiden, wird vorgeschlagen, daß der Umfangsrand der Becherwand an der Erhebung und/oder an der Gefäßseitenwand im wesentlichen abdichtend anliegt, und zwar spätestens dann, wenn beim Aufsetzen des Gefäßdeckels auf das Probenaufnahmegefäß die Erhebung in den Probenaufnahmebecher eindringt. Es wird dann das gesamte Probenmaterial ausschließlich durch die Sieböffnungen gepreßt. Man kann jedoch auch einen die lichte Weite der Sieböffnungen im wesentlichen nicht überschreitenden Abstand zwischen dem Umfangsrand der Becherwand und der Erhebung bzw. der Gefäßseitenwand vorsehen.

Aus der PCT-A-WO83/01 194 ist es bekannt, am Gefäßdeckel sowohl ein den Gefäßquerschnitt im wesentlichen ausfüllendes Sieb als auch einen zum Gefäßboden hin offenen Probenaufnahmebecher mit Öffnungen in der Becherwand anzubringen. Der Probenaufnahmebecher ist über eine Halterung am Sieb angebracht.

Ein Ziel der Erfindung liegt demgegenüber darin, ein Probenaufnahmegefäß dieser Art bereitzustellen, welches bei einfachster Handhabung eine sehr gute Verteilung des Probenmaterials in der Flüssigkeit sicherstellt. Der gemäß Anspruch 10 ausgestaltete Probenaufnahmebecher hat zusätzlich zur Funktion des Probensammelns auch noch die der feinen, vollständigen Verteilung des Probenmaterials in der Flüssigkeit. Dies wird dadurch erreicht, daß beim Zuschrauben des Gefäßes das pastöse Probenmaterial praktisch vollständig aus dem Probenaufnahmebecher herausgedrückt wird, und zwar in, der Sieböffnungsweite im Querschnitt entsprechenden, feinen Strahlen (jets), wobei je nach Art des pastösen Probenmaterials auch eine Zerkleinerung größerer Materialpartikel durch das Sieb stattfindet. Diese Materialproben-Strömungs-.fäden oberhalb des Siebs mit entsprechend großer, für die Vermischung wesentlicher Strömungsfädenoberfläche, bilden bereits eine relativ feine Probenmaterialverteilung innerhalb der Flüssigkeit. Zur weiteren Verbesserung der Verteilung kann durch entsprechende Auf- und Abbewegung des Gefäßdeckels das von der Becherwand gebildete Sieb innerhalb der Flüssigkeit auf- und abbewegt werden, wobei sich wiederum durch die einzelnen Sieböffnungen sowie durch den Ringspalt die Mischung aus Probenmaterial und Suspendierflüssigkeit in Form feiner Strömungsfäden hindurchbewegt, mit dem Ergebnis einer intensiven gleichmäßigen, feinen Durchmischung des Probenmaterials mit der Suspendierflüssigkeit. Auf Grund der Inkompressibilität der Flüssigkeit und der Feinheit der Strömungsfäden und der Wirbel bleibt bei dem Anheben und Absenken des Gefäßdeckels der Flüssigkeitspegel praktisch vollkommen ruhig. Die Gefahr der Kontamination der Umgebung durch Spritzer ist vernachlässigbar.

Die US-A 4 032 437 zeigt ein Probenaufnahmegefäß mit einem Gefäßdeckel sowie einem Gefäßboden mit zum Gefäßdeckel hin offenem Probenaufnahmebecher. Im Gegensatz zum gattungsgemäßen Probenaufnahmegefäß mit am Gefäßdeckel gehaltertem Probenaufnahmebecher und dementsprechend einfacher Probenaufnahme ohne Hilfsmittel, muß bei dem Probenaufnahmegefäß gemäß dem US-Patent mit Hilfe eines gesonderten Spatels oder dergleichen das pastöse Probenmaterial in den Probenaufnahmebecher gestrichen werden, was die Handhabung erschwert und, bei Stuhlmaterial, die Unannehmlichkeit der Probennahme durch den Patienten wesentlich erhöht. Ein mit Sieböffnungen versehener Kolben mit Stielhalterung kann, vom Deckel unabhängig, in das Gefäß eingeführt werden, jedoch nur bis zum Oberrand des Probenaufnahmebechers. Das pastöse Probenmaterial innerhalb des Probenaufnahmebechers kann daher vom Kolben gar nicht erreicht werden, so daß, im Gegensatz zur Erfindung, das pastöse Probenmaterial durch Niederdrücken des siebartigen Kolbens gar nicht aus dem Probenaufnehmebecher in den Gefäßinnenraum oberhalb des siebartigen Kolbens zur Durchmischung der Suspendierflüssigkeit gedrückt werden kann. Der Kolben gemäß dem US-Patent ist in Gleitpassung in das Gefäß eingepaßt, wohingegen bei der Erfindung ein Ringspalt zwischen dem Umfangsrand der Becherwand und der Gefäßseitenwand wenigstens im Bereich der Erhebung vorgesehen ist, welcher zusätzlich für intensive Durchmischung sorgt. Schließlich ergibt sich auf Grund der erfindungsgemäßen Halterung der Becherwand am Deckel zum einen eine Reduzierung der Anzahl der erforderlichen Bauelemente und zum anderen eine Erhöhung der Zuverlässigkeit in der Handhabung, da beim Schließen des Deckels und anschließendem Wiederöffnen des Deckels selbsttätig das Austreiben des pastösen Probenmaterials aus dem Aufnahmebecher sowie die Durchmischung mit der Suspendierflüssigkeit erfolgt.

Aus der DE-A-32 18 079 ist ein Rotor innerhalb eines Bechers bekannt, welcher durch Drehung mit ausreichend hoher Geschwindigkeit Zellanhäufungen im Bereich zwischen Rotor und Becher mit Hilfe entsprechender Scherkräfte aufbricht. Der lichte Abstand zwischen Rotor und Becher beträgt 4 mm. Bei der Erfindung dagegen, wird eine Durchmischung durch Axialverschiebung des mit Sieböffnungen versehenen Probenaufnahmebechers innerhalb des Gefäßes erzielt.

Die Erfindung wird im folgenden an Hand der Zeichnung an bevorzugten Ausführungsbeispielen erläutert. Es zeigt:

Fig. 1 eine seitliche Schnittansicht einer ersten erfindungsgemäßen Ausführungsform eines Probenaufnahmegefäßes vor dem Aufsetzen des Gefäßdeckels;

Fig. 2 die Anordnung nach Fig. 1 mit aufgesetztem Gefäßdeckel;

Fig. 3 das Probenaufnahmegefäß gemäß Fig. 1 und 2 vor dem Aufsetzen eines Filterkörpers sowie eines Filtratgefäßes;

Fig. 4 das mit dem Filterkörper und dem Filtratgefäß versehene, zum Filtrieren verwendete Probenaufnahmegefäß gemäß Fig. 3;

Fig. 5 eine seitliche Schnittansicht einer zweiten Ausführungsform eines Probenaufnahmegefäßes mit teilweise eingeschraubtem Gefäßdeckel;

Fig. 6 eine Filtrieranordnung ähnlich Fig. 4;

Fig. 7 das Detail A in Fig. 6 und

Fig. 8 einen vergrößerten Ausschnitt eines in der Anordnung gemäß Figuren 6 und 7 eingesetzten Filtergewebes (Blickrichtung B in Fig. 6).

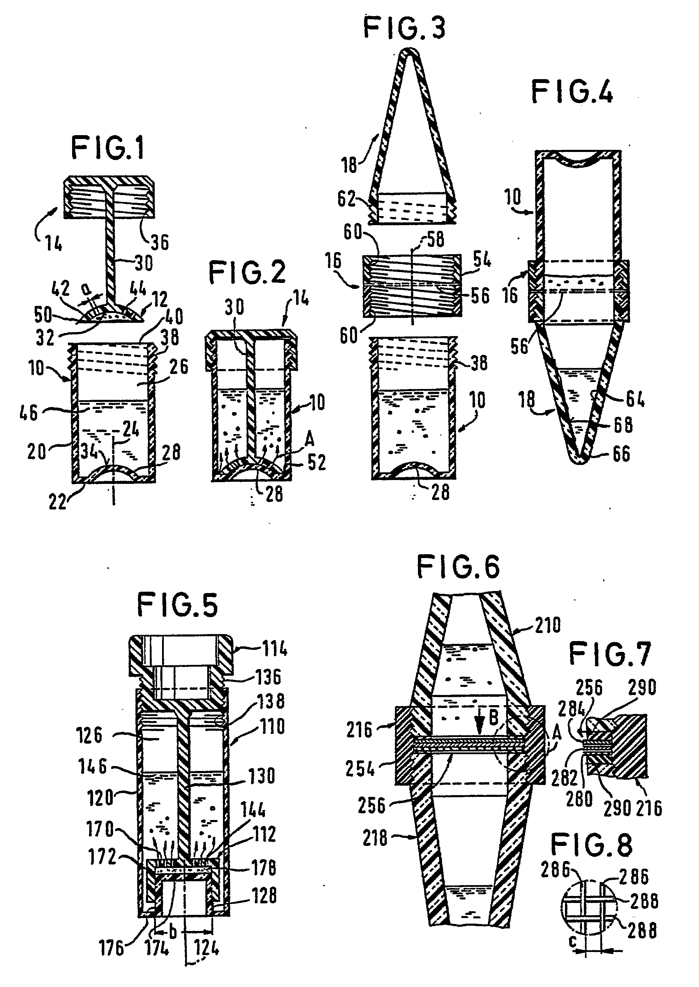

Das in den Figuren 1 bis 4 dargestellte Probenaufnahmegefäß 10 kann wahlweise mit einem in den Figuren 1 und 2 erkennbaren, einen Probeaufnahmebecher 12 aufweisenden Gefäßdeckel 14 oder, ohne daß der Gefäßinhalt umzuschütten ist, mit einem Filterkörper 16 zum Filtrieren des Gefäßinhalts entsprechend Figuren 3 und 4 verwendet werden. Das Probenaufnahmegefäß 10 weist eine hohlzylindrische Gefäßseitenwand 20 sowie einen Gefäßboden 22 auf. Der Gefäßboden 22 ist mit einer zur Gefäßachse 24 zentrisch angeordneten, vom Gefäßinnenraum 26 ausgesehen konvexen, im wesentlichen kalottenförmigen Erhebung 28 ausgeformt. Der über einen Stiel 30 mit dem Gefäßdeckel 14 verbundene Probenaufnahmebecher 12 ist komplementär zur Erhebung 28 gewölbt. Die Länge des Stiels 30 ist nun derart festgelegt, daß bei vollständig aufgeschraubtem Gefäßdeckel 14 gemäß Fig. 2 die Becherinnenseite 32 vollflächig an der Oberseite 34 der Erhebung 28 anliegt. Der angenähert hutförmige Deckel 14 ist mit einem Innengewinde 36 versehen, welches auf ein Außengewinde 38 der Becherseitenwand 20 im Bereich der Becheröffnung 40 aufschraubbar ist.

Die Becherwand 42 des Probenaufnahmebechers 12 ist, über die gesamte Wand verteilt, mit Sieböffnungen 44 versehen mit einer lichten Weite a zwischen 0,5 und 2 mm, am besten etwa 1 mm.

Zur Entnahme einer Probe wird entweder mit Hilfe eines Spatels oder dergl. die Probe in den Probenaufnahmebecher 12 gestrichen oder die Probe unmittelbar mit Hilfe des Probenaufnahmebechers aufgenommen. Aufgrund des vorgegebenen Probenaufnahmebecher-Volumens können auch quantitative Messungen durchgeführt werden. Vorher ist in das Probenaufnahmegefäß 10 eine erste Flüssigkeit 46 eingefüllt worden, die als Fixier- und/oder Transportmedium dient. Im Falle einer Anwendung der MIFC-Technik besteht die erste Flüssigkeit aus Formalin-Wasser-Glycerin zuzüglich Merthiolat (Thimerosal).

Der Gefäßdeckel 14 wird mit dem Probenaufnahmebecher 12 voraus auf das Probenaufnahmegefäß 10 aufgesetzt und mit diesem verschraubt. Während dieses Schraubvorgangs nähert sich die Becherwand 42 zunehmend der Erhebung 28. Zwischen der Becherwand 20 und dem Umfangsrand 50 der Becherwand 42 ist ein schmaler Ringspalt 52 gebildet mit einer den Wert a nicht überschreitenden Spaltweite. Aufgrund dieses Ringspalts kann beim nach unten Bewegen des Probenaufnahmebechers 12 innerhalb des Probenaufnahmegefäßes 10 die erste Flüssigkeit 46 ohne weiteres am Umfangsrand 50 vorbei nach oben ausweichen. Sobald das Probenmaterial 32 jedoch den Becherboden 22 erreicht und der Probenaufnahmebecher 12 weiterhin nach unten bewegt wird (aufgrund der Aufschraubbewegung des Gefäßdeckels 14), wird es komprimiert und aus den Sieböffnungen 44 sowie dem in Fig. 2 angedeuteten Ringspalt 52 gepreßt. In Fig. 2 sind entsprechende kleine Strömungspfeile mit A bezeichnet. Entsprechend der Sieböffnungsweite a und der Anzahl der Sieböffnungen ergeben sich eine Vielzahl feiner Materialproben-Strömungsfäder (Jets) in die erste Flüssigkeit 46.

Dies führt zu einer feinen Verteilung des Probenmaterials in der ersten Flüssigkeit 46. Probenmaterialteilchen, welche größer als die Sieböffnungsweite a sind, werden, falls möglich, zwischen den beiden wie Stempel aufeinanderdrückenden Teilen-Erhebungen 28 und Becherwand 42 zerdrückt, so daß sie schließlich durch die Sieböffnungen 44 entweichen können. Diejenigen Partikel, welche aufgrund ihrer Härte nicht zerdrückbar sind, bleiben zwischen Becherwand 42 und Erhebung 28. Damit der Gefäßdeckel 14 dennoch vollständig aufgeschraubt werden kann und somit das Probenaufnahmegefäß abdichtet, ist sowohl die Becherwand 42 als auch der Stiel 30 elastisch nachgiebig ausgebildet. Dies wird durch Fertigung dieser Teile aus Polyäthylen erreicht. Alternativ oder zusätzlich kann auch die Erhebung 28 elastisch nachgiebig ausgebildet sein.

Allein durch das Zuschrauben des Gefäßdeckels 14 erreicht man also automatisch die Feinverteilung des Probenmaterials in der ersten Flüssigkeit 46. Durch anschließendes leichtes Schütteln kann man die Suspendierung des Probenmaterials in der ersten Flüssigkeit 46 noch verstärken. Falls erforderlich, kann man bei der Probenentnahme oder später im Labor den Suspendierungsgrad in einfacher Weise noch dadurch erhöhen, daß man durch Anheben und Absenken des Gefäßdeckels 14 den Probenaufnahmebecher 12 innerhalb des Probenaufnahmegefäßes auf und ab bewegt. Es ergibt sich eine intensive Durchmischung aufgrund der Wirbelbildung im Bereich der Sieböffnungen 44 sowie des Ringspalts 52. Es werden keine zusätzlichen, ggf. eigens zu sterilisierenden Umrühr-Geräte benötigt. Die Gefahr der Kontamination der Umgebung durch Spritzer oder Lösungsmitteldämpfe ist stark reduziert.

Das Probenaufnahmegefäß 10 kann unmittelbar als Transportgefäß zwischen Probenentnahmeort und Labor verwendet werden. Die erste Flüssigkeit 46 verhindert ein Gären des Probenmaterials, so daß es nicht zu einer Explosion des Probenaufnahmegefäßes kommen kann. Ferner unterbindet die erste Flüssigkeit 46 auch eine Geruchsentwicklung bei entsprechendem Probenmaterial. Schließlich kann die erste Flüssigkeit auch für eine Sterilisierung und Fixierung des Probenmaterials sorgen.

Im Labor wird der Gefäßdeckel 14 abgenommen, ggf. eine zweite Flüssigkeit, insbesondere organisches Lösungsmittel (Äther oder Äthylacetat) oder Farbmittel (z.B. Lugolsche Lösung), zugegeben und nochmals durch Auf- und Abbewegung des Probenaufnahmebechers intensiv vermischt. Nunmehr wird zur anschließenden Filtrierung der in den Figuren 3 und 4 erkennbare Filterkörper 16 aufgeschraubt. Der Filterkörper 16 umfaßt einen hohlzylindrischen Filterträger 54, in dessen Durchgang mit Abstand zu beiden Durchgangsenden ein ein- oder mehrlagiger Filter 56 vorgesehen ist. In Richtung der Hohlzylinderachse 58 beidseits des Filters 56 ist je ein Einschraubgewinde 60 vorgesehen zur Verbindung des Filterkörpers 16 mit dem Probenaufnahmegefäß (Außengewinde 38) bzw. dem Filtratgefäß 18 (Außengewinde 62). Das Filtratgefäß 18 kann also vor oder nach dem Aufschrauben des Filterkörpers 16 auf das Probenaufnahmegefäß 10 mit dem Filterkörper 16 verschraubt werden. Der Filterträger 16 kann in besonders einfacher Weise dadurch hergestellt werden, daß man zwei jeweils mit dem Einschraubgewinde 60 versehene Schraubringe miteinander stirnseitig verklebt unter Zwischenlage des Filters 56. Ein spezieller Filteraufbau wird nachfolgend an Hand der Figuren 6 bis 8 noch näher beschrieben.

Nach dem erfolgten Zusammenschrauben der Teile 10, 16 und 18 wird die Anordnung um 180° um eine horizontale Achse in die Lage gemäß Fig. 4 gedreht. Schüttelt man bei der MIFC-Methode zum Parasitennachweis nun die Anordnung in vertikaler Richtung (ca. 15 Sekunden), so erhält man etwa die Hälfte der Suspension als Filtrat im Filtratgefäß 18. Die Filtrierung der restlichen Suspension erreicht man in einfacher Weise dadurch, daß man das obere Ende der Anordnung (d.H. das Probenaufnahmefäß 10) ergreift und zwei-bis dreimal nach abwärts gerichtet schüttelt (wie bei einem Zurückschlagen eines Quecksilber-Fieberthermometers). Es befindet sich nun die gesamte filtrierte Suspension im Filtratgefäß 18. Nun kann das als Einsendegefäß dienende Probenaufnahmegefäß 10 entfernt werden; die auf dem Filter 56 abgelagerten Partikel können gesondert untersucht werden. Die filtrierte Suspension im Filtratgefäß 18 kann nun entsprechenden Untersuchungen zugeführt werden. Im Falle der MIFC-Methode für Parasitologie läßt man dann, wenn keine Zentrifuge vorhanden ist, die Suspension ca. 12 bis 24 Stunden stehen. Falls eine Zentrifuge vorhanden ist, kann man das Filtratgefäß 18 unmittelbar in die Zentrifuge einsetzen und zentrifugieren. In beiden Fällen erhält man dann eine Flüssigkeitsschichtung im Filtratgefäß 18. Bei der MIFC-Methode interessiert lediglich die unterste Schicht innerhalb des sich nach unten konisch verjüngenden Filtratgefäßes 18. Die darüberliegenden Schichten werden vorsichtig dekantiert. Um hierbei ein versehentliches Entweichen auch der interessierenden untersten Schicht zu verhindern, oder zumindest zu erschweren, ist die Gefäßinnenseite 64 zumindest im Bereich der Spitze 66 aufgerauht und/oder mit einer die Haftung des Probenmaterials bzw. Filtrats an der Gefäßinnenseite 64 verbessernden Beschichtung versehen. Besonders bewährt hat sich eine Silikat-Beschichtung 68.

Die verbleibende unterste Schicht wird nunmehr näher untersucht. Hierzu wird ein Tropfen dieser Schicht auf einen Objektträger gebracht, ein Glasdeckel aufgesetzt und mikroskopisch untersucht.

Bei der in Fig. 5 dargestellten zweiten Ausführungsform eines Probenaufnahmegefäßes sind diejenigen Bauelemente, welche ihrer Funktion nach solchen der Ausführungsform gemäß Figuren 1 bis 4 entsprechen, mit denselben Bezugsziffern, jeweils vermehrt um die Zahl 100, versehen. Das demzufolge mit 110 bezeichnete Probenaufnahmegefäß ist im Unterschied zur ersten Ausführungsform mit einem Innengewinde 138 versehen, in welches ein dementsprechend mit einem Außengewinde 136 versehener Gefäßdeckel 114 einschraubbar ist. Der Hauptunterschied zur ersten Ausführungsform liegt jedoch in der unterschiedlichen Gestaltung des Probenaufnahmebechers 112 und dementsprechend der Erhebung 128. Der Probenaufnahmebecher ist mit einem in Bezug auf die Gefäßachse 124 radialen, kreisrunden Becherboden 170 versehen, in welchem die Sieböffnungen 144 ausgeformt sind. Vom Umfangsrand des Becherbodens 170 geht eine hohlzylindrische Becherseitenwand 172 aus. Die hierzu komplementär ausgeformte Erhebung 128 weist demnach einen Erhebungsboden 174 sowie eine hohlzylindrische Erhebungsseitenwand 176 auf, deren Außendurchmesser b etwa dem Innendurchmesser der Becherseitenwand 172 entspricht. Zwischen dem Außenumfang der Becherseitenwand 172 und dem Innenumfang der Gefäßseitenwand 120 befindet sich ein ausreichend großer Spalt, z.B. von etwa 1,5 mm, für das Überströmen der Flüssigkeit 146 beim Nachuntenschieben des Probenaufnahmebechers 112. Der vom Becherboden und von der Becherseitenwand 172 umschlossene Probenaufnahmeraum 178 ist folglich zylindrisch. Beim Einschieben des Probenaufnahmebechers 112 in das Probenaufnahmegefäß 110 gelangt schließlich die Becherseitenwand 172 in Kontakt mit der Erhebungsseitenwand 176. Die Erhebung 128 fährt in der Folge nach Art eines Kolbens in den Probenaufnahmebecher 112 ein und verdrängt das Probenmaterial innerhalb des Probenaufnahmeraums 178. Dieses wird jet-artig durch die Sieböffnungen 144 in die erste Flüssigkeit 146 gepreßt. Bei vollständig aufgeschraubtem Deckel 114 liegt der Becherboden 170 am Erhebungsboden 174 vollflächig an, es sei denn, daß Partikel, wie z.B. Steinchen, den Becherboden vom Erhebungsboden in entsprechendem Abstand halten. Da der Stiel 130 elastisch nachgiebig ist, kann dennoch der Gefäßdeckel 114 vollständig abdichtend aufgeschraubt werden.

Zur Verbesserung der Verteilung des Probenmaterials in der jeweiligen Flüssigkeit innerhalb des Probenaufnahmegefäßes 110 kann man, wie schon an Hand der Figuren 1 und 2 beschrieben, den Probenaufnahmebecher 112 innerhalb des Gefäßinnenraums 126 mehrfach auf und ab schieben.

In einer nicht dargestellten Ausführungsform der Erfindung ist der den Probenaufnahmebecher tragende Stiel lösbar mit dem Gefäßdeckel verbunden, wozu der Gefäßdeckel an seiner Innenseite dementsprechend mit einem Einsteck-Sackloch versehen sein kann.

Der genauere Siebaufbau geht aus den Figuren 6 bis 8 hervor. Bauelemente, welche ihrer Funktion nach solchen in den Figuren 1 bis 3 entsprechen, sind mit denselben Bezugsziffern, jeweils vermehrt um die Zahl 200, versehen. In den Filterträger 216 ist von einer Seite aus das Probenaufnahmegefäß 210 eingeschraubt und von der anderen Seite aus das Filtratgefäß 218. Im nunmehr zu beschreibenden Ausführungsbeispiel haben Filtratgefäß 218 und Probenaufnahmegefäß 210 die gleiche spitz zulaufende Form, da die erfindungsgemäße Filtrierung innerhalb eines geschlossenen Systems auch unabhängig von der Probensuspendierung mit Hilfe entsprechend ausgebildetem Probenaufnahmegefäß und Gefäßdeckel (Erhebung bzw. Probenaufnahmebecher) durchgeführt werden kann, obschon sie, insbesondere bei der MIFC-Technik, mit besonderem Vorteil mit dementsprechend ausgebildetem Probenaufnahmegefäß durchführbar ist.

Der Filter 256 ist insgesamt drei-lagig. Auf ein Stützsieb 280 folgt ein Hauptfilter 282 und anschließend ein Vorfilter 284. Wenigstens eine der Lagen, am besten sämtliche Lagen, werden jeweils von einem Gewebesieb gebildet, also aus einem Sieb mit gewebeartig sich gegenseitig kreuzenden Gewebefäden oder -strängen. In Fig. 8 sind parallel zueinander liegende erste Fäden 286 gezeigt, welche sich rechtwinkelig mit zweiten Fäden 288 kreuzen. Die lichte Maschenweite c zwischen aufeinanderfolgenden Fäden ist entsprechend der jeweiligen Lage (Stützsieb bzw. Hauptfilter bzw. Vorfilter) und der gewünschten, gerade noch durchzulassenden Teilchengröße festgelegt. So wird man beispielsweise zum Abfiltrieren von Bakterien, Viren, Enzymen und Substraten von größeren Parasiten folgende Maschenweiten wählen: Stützsieb 20 - 100 µm; Hauptfilter 3 - 5 µm; Vorfilter 20 - 100µm. Zum Trennen von Bakterien und Pilzsporen von größeren Partikeln wählt man für die lichte Maschenweite c folgende Werte: Stützsieb 20 - 100 µm; Hauptfilter 0,15 - 0,45 µm; Vorfilter 5 - 20 µm.

Die aus Metall und/oder Kunststofffäden bzw. -strängen gebildeten Gewebefäden verhindern ein Anhaften von Partikeln am Filter, welche an und für sich den Filter passieren sollten. Auch ergibt sich aufgrund der Gewebestruktur eine hohe Trennschärfe (geringe Streuung der lichten Maschenweite bei einem Gewebesieb).

Um die drei Lagen 280 bis 284 zusammenzuhalten und zudem ein Austreten von Flüssigkeit aus der zusammengeschraubten Anordnung der Teile 210, 216, 218 zu verhindern, ist beidseits des drei-lagigen Filters 265 jeweils ein O-Ring 290 eingesetzt, welcher gemäß Fig. 7 rechteckigen Querschnitt aufweist. Mit seinem Außenumfang liegt jeder Ring 290 am Innenumfang des hohlzylindrischen Filterträgers 254 an. An die vom drei-lagigen Filter 256 jeweils abgewandte Stirnseite drückt das Filtratgefäß 218 bzw. das Probenaufnahmegefäß 210. Der mehrteilige Filter 256 kann austauschbar ausgebildet sein, um einen Filterträger 216 durch entsprechende Filterwahl an die verschiedenen Anwendungsbereiche anpassen zu können.

Die Handhabung der Anordnung gemäß Fig. 6 entspricht der der Anordnung gemäß Figuren 3 und 4. Auf das die zu filtrierende Suspension enthaltende Probenaufnahmegefäß 210 ist also der Filterträger 216 samt Filtratgefäß 218 aufzuschrauben und anschließend um 180° um eine horizontale Achse zu drehen. Das anschließende Filtrieren kann durch Schütteln oder Schlagen oder Zentrifugieren der Anordnung gemäß Fig. 6 unterstützt werden. Aufgrund des vollständig abgeschlossenen Systems ist die Kontaminationsgefahr (Spritzer, Lösungsmitteldämpfe oder dergl.) beseitigt. Auch entfällt jedwede Geruchsbelästigung. Die Verarbeitung von unangenehme Gefühle auslösenden Materialien, wie z.B. Stuhlproben, gestaltet sich auf die angegebenen Weise wesentlich angenehmer.

Die erfindungsgemäße Filtrier-Anordnung eignet sich zur schnellen Reinigung oder Steril-Filtration von sämtlichen flüssigen Suspensionen oder Lösungsmitteln, wobei keine nennenswerten Verluste an Probenmaterial auftreten. Für die erfindungsgemäße Filtrieranordnung kann unmittelbar das Transportgefäß bzw. Verarbeitungsgefäß bzw. Lagerungsgefäß verwendet werden, da lediglich der Filterträger samt Filtratgefäß in üblicher Weise, z.B. durch Aufschrauben, aufzusetzen ist. Die Filtriermethode eignet sich zur Anwendung bei empfindlichen Chromatografiesystemen, wie z.B. HPLC, im Bereich der Immunologie, Molekularbiologie, Zellbiologie, Bakteriologie, Virologie oder dergl.

## Patentansprüche

1. Probenaufnahmegefäß (10) für in Flüssigkeit, insbesondere Suspendierflüssigkeit zu verteilendes pastöses Probenmaterial, insbesondere Stuhl, mit einem an einem Gefäßdeckel (14) angebrachten, zum Gefäßboden (22) hin offenen Probenaufnahmebecher (12) im Gefäßinnenraum (26), dadurch gekennzeichnet, daß die Becherwand (42; 170 ,172) wenigstens bereichsweise mit Sieböffnungen (44; 144) versehen ist, daß der Gefäßboden (22) mit einer Erhebung (28; 128) ausgebildet ist, deren dem Gefäßinnenraum (26; 126) zugewandte Oberseite (34) im wesentlichen komplementär zur Becherinnenseite (32) geformt ist, und daß die Becherinnenseite (32) bei geschlossenem Gefäß (10) an der Oberseite (34) der Erhebung (28; 128) im wesentlichen vollflächig anliegt.

2. Probenaufnahmegefäß nach Anspruch 1, dadurch gekennzeichnet, daß die erhebungsseitigen Sieböffnungsränder der Becherwand (42) scharfkantig sind.

3. Probenaufnahmegefäß nach Anspruch 1 oder 2, dadurch gekennzeichnet, daß bei Stuhlproben die Sieböffnungen eine lichte Weite (a) von etwa 0,5 bis 2 mm, vorzugsweise etwa 1 mm aufweisen.

4. Probenaufnahmegefäß nach einem der vorhergehenden Ansprüche, dadurch gekennzeichnet, daß der Probenaufnahmebecher (12; 112) am Gefäßdeckel (14; 114) in Richtung zum Deckel hin nachgiebig gehaltert ist.

5. Probenaufnahmegefäß nach einem der vorhergehenden Ansprüche, dadurch gekennzeichnet, daß die Becherwand (42) und/oder die Erhebung nachgiebig ausgebildet sind.

6. Probenaufnahmegefäß nach Anspruch 4 oder 5, dadurch gekennzeichnet, daß eine vorzugsweise stielförmige Halterung (30; 130) für den Probenaufnahmebecher (42) am Gefäßdeckel (14; 114) und/oder die Becherwand (42) und/oder die Erhebung mit Polyäthylen ausgebildet sind.

7. Probenaufnahmegefäß nach einem der vorhergehenden Ansprüche, dadurch gekennzeichnet, daß die Becherwand (42) im wesentlichen kalottenförmig gekrümmt ist.

8. Probenaufnahmegefäß nach einem der Ansprüche 1 bis 6, dadurch gekennzeichnet, daß die Becherwand von einem mit Sieböffnungen (144) versehenen Becherboden (170) sowie einer vom Becherboden (170) ausgehenden, im wesentlichen hohlzylindrischen Becherseitenwand (172) gebildet ist.

9. Probenaufnahmegefäß nach einem der vorhergehenden Ansprüche, dadurch gekennzeichnet, daß beim Aufsetzen des Gefäßdeckels (14; 114) auf das Probenaufnahmegerät (10; 110), spätestens dann, wenn die Erhebung (28; 128) in den Probenaufnahmebecher (12; 112) eindringt, der Umfangsrand (50) der Becherwand (42) an der Erhebung (128) und/oder an der Gefäßseitenwand (20) im wesentlichen abdichtend anliegt oder zur Erhebung (128) bzw. zur Gefäßseitenwand (20) eine die lichte Weite (a) der Sieböffnungen im wesentlichen nicht überschreitenden Abstand einhält.

10. Probenaufnahmegefäß für in Flüssigkeit, insbesondere Suspendierflüssigkeit, zu verteilendes pastöses Probenmaterial, insbesondere Stuhl, mit einem Gefäßdeckel, an welchem ein den Gefäßquerschnitt im wesentlichen ausfüllendes Sieb mit lichter Sieböffnungsweite zwischen 0,5 und 2 mm sowie über eine Halterung ein zum Gefäßboden hin offener Probenaufnahmebecher mit Öffnungen in der Becherwand angebracht ist, insbesondere nach einem der Ansprüche 1–9, dadurch gekennzeichnet, daß das Sieb von der Becherwand (42; 170, 172) gebildet ist, daß der Gefäßboden (22) mit einer Erhebung (28; 128) ausgebildet ist, deren dem Gefäßinnenraum (26; 126) zugewandte Oberseite (34) im wesentlichen komplementär zur Becherinnenseite (32) geformt ist, daß die Becherinnenseite (32) bei geschlossenem Gefäß (10) an der Oberseite der Erhebung (28; 128) im wesentlichen vollflächig anliegt, und daß wenigstens im Bereich der Erhebung (28; 128) zwischen dem Umfangsrand der Becherwand und der Gefäßseitenwand (20) ein Ringspalt (52) gebildet ist und mit einer die lichte Weite (a) der Sieböffnungen nicht überschreitenden Spaltweite.

**Claims**

1. Sample-taking vessel (10) for pasty sample material, especially faeces, to be distributed in fluid, especially suspension fluid, having a sample-taking cup (12) fitted on a vessel lid (14) and open towards the bottom (22) of the vessel, in the interior space (26) of the vessel, characterised in that the cup wall (42; 170, 172) is provided, at least by zones, with sieve openings (44; 144), in that the vessel bottom (22) is formed with a raised portion (28; 128) of which the upper side (34) facing the interior space (26; 126) of the vessel is shaped substantially complementarily to the inner side (32) of the cup, and in that the cup inner side (32) lies with substantially the whole surface on the upper side (34) of the raised portion (28; 128), when the vessel (10) is closed.

2. Sample-taking vessel according to Claim 1, characterised in that the edges of the sieve openings of the cup wall (42) on the side towards the raised portion are sharp-edged.

3. Sample-taking vessel according to Claim 1 or 2, characterised in that in the case of faeces samples the sieve openings have a clear internal width (a) of about 0.5 to 2 mm, preferably about 1 mm.

4. Sample-taking vessel according to one of the preceding Claims, characterised in that the sample-taking cup (12; 112) is retained on the vessel lid (14; 114) yieldably in the direction towards the lid.

5. Sample-taking vessel according to one of the preceding Claims, characterised in that the cup wall (42) and/or the raised portion are made yieldable.

6. Sample-taking vessel according to Claim 4 or 5, characterised in that a mounting (30; 130) preferably of stick form for the sample-taking cup (42) on the vessel lid (14; 114) and/or the cup wall (42) and/or the raised portion are formed with polyethylene.

7. Sample-taking vessel according to one of the preceding Claims, characterised in that the cup wall (42) is curved in substantially part-spherical form.

8. Sample-taking vessel according to one of Claims 1 to 6, characterised in that the cup wall is formed by a cup bottom (170) provided with sieve openings (144) and a substantially hollow-cylindrical cup side wall (172) issuing from the cup bottom (170).

9. Sample-taking vessel according to one of the preceding Claims, characterised in that in the setting of the vessel lid (14; 114) upon the sample-taking apparatus (10; 110), at the latest when the raised portion (28; 128) penetrates into the sample-taking cup (12; 112), the circumferential edge (50) of the cup wall (42) rests in substantially sealing manner on the raised portion (128) and/or on the vessel side wall (20), or maintains from the raised portion (128) or from the vessel side wall (20) a spacing not substantially exceeding the clear internal width (a) of the sieve openings.

10. Sample-taking vessel for pasty sample material, especially faeces, to be distributed in fluid, especially suspension fluid, with a vessel lid on which there are fitted a sieve, substantially filling out the vessel cross-section, with clear internal width of the sieve openings between 0.5 and 2 mm and, through a mounting fitting, a sample-taking cup, open towards the vessel bottom, with openings in the cup wall, especially according to one of Claims 1-9, characterised in that the sieve is formed by the cup wall (42; 270, 172), in that the vessel bottom (22) is formed with a raised portion (28; 128) of which the upper side (34) facing the interior space (26; 126) of the vessel is shaped substantially complementarily to the inner side (32) of the cup, in that the inner side (32) of the cup rests with substantially its whole surface on the upper side of the raised position (28; 128) when the vessel (10) is closed, and in that at least in the region of the raised portion (28; 128) between the circumferential edge of the cup wall and the side wall (20) of the vessel an annular gap (52) is formed with a gap width not exceeding the clear internal width (a) of the sieve openings.

**Revendications**

1. Eprouvette pour échantillon d'une matière pâteuse, plus particulièrement de selles, devant être mélangé dans un liquide, plus particulièrement dans une suspension, comprenant un gobelet (10) et un couvercle (14) muni à sa partie inférieure d'un godet (12) de prise d'échantillon, s'étendant à l'intérieur du gobelet (10) jusqu'à son fond (22), caractérisée en ce que la paroi (42, 170, 172) du godet (12) est munie d'orifices (44) filtrants, et en ce que le fond (22) du gobelet (10) comprend une proéminence (28, 128) dont la surface supérieure (34) est essentiellement complémentaire à la surface inférieure (32) du godet (12) de manière à ce que, lorsque l'éprouvette est fermée, la surface inférieure (32) du godet (12) adhère parfaitement à la surface supérieure (34) de la proéminence (28, 128) du fond (22) du gobelet (10).

2. Eprouvette suivant la revendication 1, caractérisée en ce que les bords des orifices (44) filtrants dirigés vers la proéminence (28, 128) sont tranchants.

3. Eprouvette suivant l'une des revendications 1 ou 2, caractérisée en ce que les orifices (44) filtrants, pour les échantillons de selles, ont un faible diamètre (a), variant de 0,5 à 2 mm, et de préférence d'environ 1 mm.

4. Eprouvette suivant l'une des revendications précédentes, caractérisée en ce que le godet (12, 112) de prise d'échantillon est fixé au couvercle (14, 114) d'une manière flexible.

5. Eprouvette suivant l'une des revendications précédentes, caractérisée en ce que la paroi (42) du godet (12) et/ou la proéminence (28, 128) sont fabriquées en matière plus ou moins flexible.

6. Eprouvette suivant l'une des revendications 4 ou 5, caractérisée en ce que le support (30, 130), de préférence en forme de tige, servant à la fixation du godet (42) de prise d'échantillon au couvercle (14, 114), et/ou la paroi (42) du godet (12) et/ou la proéminence (28, 128) sont construits au moyen de polyéthylène.

7. Eprouvette suivant l'une des revendications précédentes, caractérisée en ce que la paroi (42), du godet (12) est essentiellement courbée en forme de calotte sphérique.

8. Eprouvette suivant l'une des revendications 1 à 6, caractérisée en ce que la paroi (172) du godet comprenant un fond (170) muni d'orifices filtrants (144) et une paroi latérale relevée sur ce fond (170), possède une forme essentiellement cylindrique creuse.

9. Eprouvette suivant une des revendications précédentes, caractérisée en ce qu'au moment du dépôt du couvercle (14, 114) sur le gobelet (10, 110) et au plus tard, au moment où la proéminence (28, 128) pénètre dans le godet (12, 112) de prise d'échantillon, le bord annulaire (50) de la paroi (42) du godet (12) assure un joint essentiellement étanche avec la proéminence (28, 128) et/ou à la paroi (20) du gobelet, ou tout au moins comprend un intestice avec la paroi (20) du gobelet non supérieur au diamètre (a) des orifices filtrants (44, 144).

10. Eprouvette pour échantillon d'une matière pâteuse, plus particulièrement de selles, devant être mélangé dans un liquide, plus particulièrement une suspension, comprenant un gobelet muni d'un couvercle auquel est fixé essentiellement un filtre dont la dimension occupe la section du gobelet, et comprenant des orifices filtrants dont le diamètre est compris entre 0,5 et 2 mm, et qui par l'intercession d'un support comprend un godet pour la prise d'échantillon, s'étendant jusqu'au fond du récipient et ayant des orifices dans sa paroi, plus particulièrement suivant une des revendications 1 à 9, caractérisée en ce que le filtre fait partie de la paroi (42, 170, 172) du godet, et en ce que le fond (22) du gobelet (10) est muni d'une proéminence (28, 128) de manière à ce que sa face supérieure (34) adjacente à l'enceinte intérieure (26, 126) du gobelet a une forme essentiellement complémentaire à la face inférieure (32) du godet, de manière à ce que lorsque l'éprouvette est fermée, cette face inférieure (32) vienne couvrir entièrement la face supérieure de la proéminence (28, 128), et qu'au moins au niveau de la proéminence (28, 128) entre le bord du godet et la paroi cylindrique (20) du gobelet il y ait un espace annulaire (52) dont la largeur ne soit pas supérieure au diamètre (a) des orifices filtrants (44, 144).

EP 0 223 878 B1

FIG.1

FIG.2

FIG.3

FIG.4

FIG.5

FIG.6

FIG.7

FIG.8